Europäisches Patentamt

European Patent Office

Office européen des brevets

(11) Veröffentlichungsnummer: **0 240 826 B1**

(12) **EUROPÄISCHE PATENTSCHRIFT**

(45) Veröffentlichungstag der Patentschrift: **23.10.91**

(21) Anmeldenummer: **87104258.6**

(22) Anmeldetag: **23.03.87**

(51) Int. Cl.5: **A61K 47/44, A23K 1/10**

(54) **Köder für Tiere.**

(30) Priorität: **03.04.86 DE 3611122**

(43) Veröffentlichungstag der Anmeldung:
**14.10.87 Patentblatt 87/42**

(45) Bekanntmachung des Hinweises auf die
Patenterteilung:
**23.10.91 Patentblatt 91/43**

(84) Benannte Vertragsstaaten:
**AT BE CH DE ES FR GB GR IT LI LU NL SE**

(56) Entgegenhaltungen:
**CH-A- 642 846**
**DE-A- 2 142 167**
**DE-A- 2 759 166**
**DE-U- 8 509 193**
**FR-A- 2 163 352**

(73) Patentinhaber: **Klocke, Hartmut**
**Industriestrasse**
**W-7504 Weingarten/Baden(DE)**

(72) Erfinder: **Schneider, Lothar G., Dr.**
**Brahmsweg 17**
**W-7400 Tübingen(DE)**
Erfinder: **Schneider, Irma**
**Brahmsweg 17**
**W-7400 Tübingen(DE)**

(74) Vertreter: **Frank, Gerhard, Dipl.-Phys.**
**Patentanwälte Dr. F. Mayer & G. Frank Westliche 24**
**W-7530 Pforzheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die Erfindung betrifft einen Köder für Tiere mit pharmazeutischem Wirkstoff, beispielsweise zur Tollwutimpfung, wobei der Wirkstoff in einer fetthaltigen Trägersubstanz enthalten ist, die außerdem einen spezifischen Lockstoff enthält, und wobei die Fettkomponente der Trägersubstanz einen Schmelzpunkt zwischen 20° und 60° Celsius aufweist.

Als Trägersubstanz, die im wesentlichen zur Anlockung der zu behandelnden Tierart dient, wurden beispielsweise bisher selbst Tiere oder Teile von Tierkörpern verwendet. Beispielsweise wurden für diese Zwecke Hühnerkopf-Köder mit entsprechenden Wirkstoffen präpariert, die dann im betroffenen Revier zur oralen Immunisierung zum Beispiel von Füchsen gegen Tollwut, ausgelegt wurden. Bei der Aufnahme der Hühnerköpfe gelangt dabei auch der Tollwut-Impfstoff an die Mundschleimhäute des Fuches und wird dort resorbiert.

Zu einem ähnlichen Zweck, nämlich dazu, Tiere zur Aufnahme einer bestimmten Nahrung zu bewegen, ist in der DE-Anmeldung B 3 658 vorgeschlagen, als fetthaltige Trägersubstanz beispielsweise Leberwurst zu verwenden, der ein spezifischer Lockstoff wie Fischmehl beigemischt ist.

Das Präparieren derartiger Köder, zum Beispiel von Tierkörper-Ködern oder der gattungsgemäßen "Leberwurst-Köder" ist jedoch nur manuell zu bewerkstelligen und daher sehr aufwendig, so daß insbesondere für großflächige Aktionen, wie Tierimpfungen, diese Methode praktisch nicht anwendbar ist.

Aufgabe der Erfindung ist es daher, einen Köder so auszubilden, daß seine großindustrielle Herstellung unter Verwendung von wirtschaftlichen Trägersubstanzen ermöglicht wird. Eine weitere Aufgabe besteht ergänzend darin, diese Trägersubstanz derart zu wählen, daß der fertige Köder weitgehend umweltstabil ist, das heißt resistent gegen Temperatur- und Feuchtigkeitseinflüsse in dem Revier, in dem er zum Einsatz kommt.

Erfindungsgemäß wird diese Aufgabe dadurch gelöst, daß die Trägersubstanz mindestens einen Zuschlagstoff zur Stabilisierung der Formhaltigkeit des Köders enthält und daß die relativen Anteile Fettkomponente/Zuschlagstoffe so gewählt sind, daß das Gemisch oberhalb des Schmelzpunktes der Fettkomponente pastös verarbeitbar und im Anwendungstemperatur-Bereich nicht brüchig ist.

Köder für Tiere in Form einer umhüllten Wirksubstanz sind beispielsweise aus der CH-A-642 846 oder dem DE-U-8 509 193 bekannt; als Trägerstoffe werden hierbei unter anderem ein eßbarer Gummi vorgeschlagen (CH-A-642 846) oder gummiartige Stoffe oder Kunststoffe oder Wachse (DE-U-8 509 193 , wo als Umhüllungsmaterial auch Polystyrol, Kork oder Kunststoffschäume in Betracht gezogen werden).

Eine bestimmte Auswahl dieser Trägersubstanzen im Hinblick auf seine Umweltstabilität ist in diesen Druckschriften nicht angesprochen.

Die erfindungsgemäße Wahl einer Mischung zwischen einer Fettkomponente und einem Zuschlagstoff zur Stabilisierung ermöglicht einerseits eine leichte Verarbeitbarkeit des Gemisches oberhalb des Schmelzpunktes der Fettkomponente (pastös), andererseits ist dadurch gewährleistet, daß nach der Abkühlung dieses Gemisches nach der Herstellung des Köders dieses erstarrt und in den praktisch auftretenden Anwendungstemperaturbereichen formstabil bleibt und insbesondere auch nicht brüchig wird, d.h. beim Zubiß des zu ködernden Wildes nicht zerbröselt oder zerbröckelt. Dieser Trägersubstanz ist ein tierartspezifischer Lockstoff beigefügt, so daß die Trägersubstanz als "Kunstköder" eine sehr attraktive "Verpackung" für die zu ködernde Tierart darstellt und diese zur Aufnahme und zum Einbiß in die Trägersubstanz verleitet, wodurch dann auch der Wirkstoff von den Mundschleimhäuten des betreffenden Tieres aufgenommen wird.

Vorteilhafterweise umgibt dabei die Trägersubstanz in Form einer Hülle eine separat abgepackte Wirkstoffeinheit vollständig. Ein derartiger Fertigköder ist besonders leicht handhabbar, wenn die Trägersubstanz mit der Wirkstoffeinheit in einer Form ausdrückbar untergebracht ist, insbesondere kann es sich hierbei um eine napfartige Tiefziehform handeln, aus der der Fertigköder tablettenartig durch Druck auf den Boden des Tiefziehnapfes ausgedrückt werden kann.

Eine bevorzugte Trägersubstanz besteht aus Pflanzenfett oder tierischem Fett als Fettkomponente und aus Fischmehl, das gleichzeitig Zuschlagstoff und Lockstoff darstellt. Das Fischmehl erfüllt zusammen mit dem Fett die oben beschriebenen Anforderungen und stellt außerdem einen sehr starken Lockstoff dar.

In weiterer Ausbildung der Erfindung ist vorgesehen, daß als Feuchtigkeitsschutz ein wachshaltiger weiterer Zusatzstoff in der Trägersubstanz enthalten ist.

Dieser wachshaltige Zusatzstoff, beispielsweise Paraffin, schützt den Fertigköder vor Feuchtigkeitseinflüssen, die zu einem Aufweichen der Trägersubstanz und damit zum Unwirksamwerden des Köders führen könnte. Der wachshaltige Zusatzstoff bewirkt dagegen in der Mischung einen Schutzüberzug ("Coating"), der zu einer Verlängerung der wirksamen Auslegedauer des Köders führt und auch eine weitere Beeinflussung der Konsistenz des Fett/Zuschlagstoff-Gemisches im obigen Sinne ermöglicht (pastös bei der Verarbeitung und stabil im Anwendungstemperaturbereich).

Der erfindungsgemäße Fertigköder ist durch geeignete Wahl der Komponenten der Trägersubstanz somit ohne Schwierigkeiten sowohl auf die anzusprechende Tierart als auch deren spezifische Umgebung, insbesondere hinsichtlich der auftretenden Temperaturen, "einstellbar". Zur Feststellung der Akzeptanz des Köders bei der betreffenden Tierart können in der Trägersubstanz auch ohne Schwierigkeiten Markierstoffe eingebracht sein, die spätere Rückschlüsse auf die Aufnahme der Köder zulassen.

Der erfindungsgemäße Köder ist industriell einfach herstellbar, erfindungsgemäß sind als Verfahrensschritte vorgesehen, daß zunächst die Fettkomponente verflüssigt wird, dann die Zumischung des Zuschlagstoffes erfolgt und dieses Gemisch dann unter die Inaktivierungstemperatur des verwendeten Wirkstoffs abgekühlt wird, sofern dies erforderlich ist, um den später einzulegenden Wirkstoff nicht zu gefährden. Dieses pastöse Gemisch wird dann portionsweise in eine Tiefziehform eingespritzt, bis deren Boden vollständig bedeckt ist und dick genug ist, um eine ausreichende Stabilität aufzuweisen. Danach wird eine abgepackte Wirkstoffeinheit in die Form auf den Boden aufgelegt und die Form wird mit dem pastösen Gemisch dann aufgefüllt, bis die eingelegte Wirkstoffeinheit vollständig bedeckt ist. Danach wird der Köder abgekühlt, wobei das Gemisch erstarrt, wobei die Erstarrungstemperatur des Gemisches durch dessen Zusammensetzung so gewählt ist, daß sie über dem Anwendungstemperaturbereich des Köders liegt, so daß gewährleistet ist, daß bei den üblicherweise im Revier auftretenden Temperaturen der Köder formstabil bleibt, ohne aber brüchig zu werden.

Das als Feuchtigkeitsschutz verwendete Paraffin wird vorzugsweise in flüssiger Form der bereits verflüssigten Fettkomponente zugegeben und mit dieser vermischt, entsprechendes gilt auch für den Lockstoff (bei der Verwendung von Fischmehl kann der Lockstoff entfallen). Für spezielle Anwendungsbereiche ist es auch möglich, anstelle der homogenen Lockstoffverteilung in der Trägersubstanz diesen nach der Fertigstellung des Köders aufzuspritzen oder aufzusprühen.

Es ist auch möglich, einen wachshaltigen Zusatzstoff, wie beispielsweise das Paraffin, nach der Fertigstellung des Köders auf diesen aufzubringen, jedoch muß hierbei darauf geachtet werden, daß die Geruchswirkung des Köders dadurch nicht beeinträchtigt wird. Bei der Zugabe des Paraffins zu der Fettkomponente bewirkt ersteres lediglich einen Überzug (Coating) dieser Komponente, ohne daß das auch als Lockstoff dienende Fischmehl dadurch in seiner Wirkung beeinträchtigt wird.

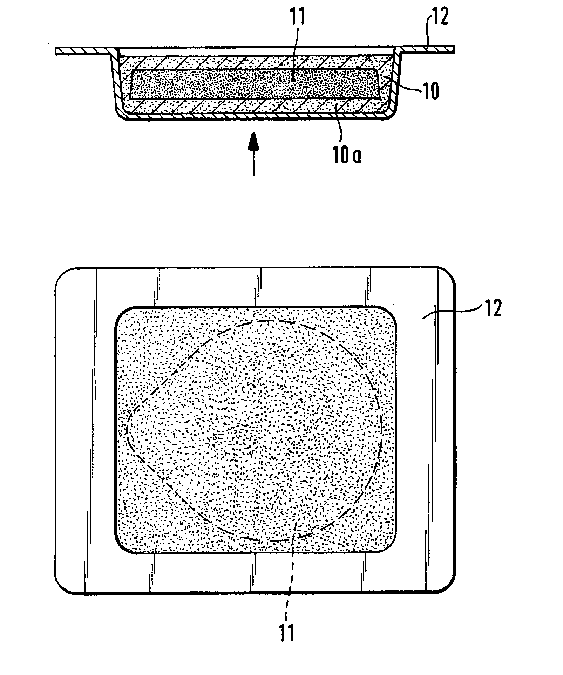

Ein Ausführungsbeispiel des erfindungsgemäßen Köders wird anhand der Zeichnung kurz erläutert:

In einem Tiefziehnapf 12 ist die Trägersubstanz als Hülle 10 eingebracht, die eine abgepackte Wirkstoffeinheit 11 umschließt, wobei die Wirkstoffeinheit 11 auf dem zunächst eingespritzten Boden 10a der Hülle 10 aufsitzt. Nach dem Einspritzen der Trägersubstanz in die Form 12 erstarrt die Trägersubstanz und der Fertigköder kann dann durch Druck auf den Boden der Form 12 (Pfeil) tablettenartig aus der Form 12 herausgedrückt werden.

Derartige Packungen können in großer Anzahl hergestellt werden und ermöglichen somit erstmalig den Einsatz von Ködern auch in großflächigen Revieren zur Durchführung wirksamer Maßnahmen gegen Tierkrankheiten oder von Tieren ausgehenden Krankheiten. Auch die Herstellungskosten lassen sich gegenüber den vorbekannten Ködern unter Verwendung von Tierteilen reduzieren.

Die Trägersubstanz wird beispielsweise wie folgt hergestellt:
Ein pflanzliches Fett mit einem Schmelzpunkt von ca. 35° C wird bei 54° C über Nacht verflüssigt, desgleichen Paraffin bei ca. 75° C.

Als Zuschlagstoff und gleichzeitig Lockstoff wird Fischmehl mit einer maximalen Korngröße von ca. 100μm bereitgehalten.

Von dem verflüssigten Pflanzenfett werden 5 Volumenteile mit 1 Volumenteil Paraffin vermischt, zu der Mischung werden 4 Teile Fischmehl unter ständigem Rühren hinzugegeben, und die Mischung auf eine Temperatur zwischen 40-43° C abgekühlt. Nach Erreichen dieses Temperaturbereichs kann mit der portionsweisen Abfüllung, wie beschrieben, begonnen werden.

## Patentansprüche

1. Köder für Tiere mit pharmazeutischem Wirkstoff, beispielsweise zur Tollwutimpfung, wobei der Wirkstoff in einer fetthaltigen Trägersubstanz enthalten ist, die außerdem einen spezifischen Lockstoff enthält, und wobei die Fettkomponente der Trägersubstanz einen Schmelzpunkt zwischen 20 bis 60° aufweist, dadurch gekennzeichnet, daß die Trägersubstanz mindestens einen Zuschlagstoff zur Stabilisierung der Formhaltigkeit des Köders enthält und daß die relativen Anteile Fettkomponente/Zuschlagstoffe derart gewählt sind, daß das Gemisch oberhalb des Schmelzpunktes der Fettkomponente pastös verarbeitbar und im Anwendungstemperatur-Bereich nicht brüchig ist.

2. Köder nach Anspruch 1, dadurch gekennzeichnet, daß die Trägersubstanz in Form einer Hülle (10) eine separat abgepackte Wirkstoffeinheit (11) vollständig umschließt.

3. Köder nach Anspruch 1 und 2, dadurch gekennzeichnet, daß die Trägersubstanz mit der Wirkstoffeinheit in einer Form (12) ausdrückbar untergebracht ist.

4. Köder nach Anspruch 1, dadurch gekennzeichnet, daß die Fettkomponente Pflanzenfett oder tierisches Fett ist.

5. Köder nach Anspruch 1, dadurch gekennzeichnet, daß als Zuschlagstoff und Lockstoff Fischmehl verwendet ist.

6. Köder nach Anspruch 1, dadurch gekennzeichnet, daß als Feuchtigkeitsschutz ein wachshaltiger weiterer Zusatzstoff in der Trägersubstanz enthalten ist.

7. Köder nach Anspruch 6, dadurch gekennzeichnet, daß der wachshaltige Zusatzstoff Paraffin ist.

8. Köder nach Anspruch 1, dadurch gekennzeichnet, daß in der Trägersubstanz Markierstoff enthalten sind.

9. Verfahren zur Herstellung eines Köders nach Anspruch 1, gekennzeichnet durch folgende Verfahrensschritte:
   a) Verflüssigung der Fettkomponente,
   b) Zumischung des Zuschlagstoffes,
   c) Abkühlung des Gemisches unter die Inaktivierungstemperatur des verwendeten Wirkstoffs (sofern erforderlich),
   d) portionsweises Einspritzen des pastösen Gemisches in eine Form, bis deren Boden vollständig bedeckt ist,
   e) Einlegen einer Wirkstoffeinheit in die Form,
   f) Auffüllung der Form, bis die Wirkstoffeinheit vollständig bedeckt ist,
   g) Abkühlung des Köders unterhalb der über dem Anwendungstemperaturbereich liegenden Erstarrungstemperatur des Gemisches.

10. Verfahren nach Anspruch 9, dadurch gekennzeichnet, daß als Zuschlagstoff Fischmehl mit einer Korngröße von unterhalb 100μm verwendet wird.

11. Verfahren nach Anspruch 9, dadurch gekennzeichnet, daß vor der Zumischung des Zuschlagstoffes der flüssigen Fettkomponente flüssiges Paraffin zugegeben und vermischt wird.

12. Verfahren nach Anspruch 9, dadurch gekennzeichnet, daß der Lockstoff dem Gemisch Fettkomponente/Zuschlagstoff zugegeben wird.

13. Verfahren nach Anspruch 9, dadurch gekennzeichnet, daß der Lockstoff auf den fertigen Köder aufgespritzt oder aufgesprüht wird.

**Claims**

1. Bait for animals, having a pharmaceutical active substance, for example for vaccination against rabies, wherein the active substance is contained in a fat-containing carrier substance which, in addition, contains a specific lure material, and wherein the fat component of the carrier substance has a melting point between 20° and 60° C, characterised in that the carrier substance contains at least one additive for stabilising the shape retention of the bait, and in that the relative proportions for the mixture of fat component and additives are selected in such a manner that the mixture is processable in its pasty form above the melting point of the fat component and is not brittle within the operational temperature range.

2. Bait according to claim 1, characterised in that the carrier substance, in the form of a covering (10), completely surrounds a separately packaged unit of active substance (11).

3. Bait according to claims 1 and 2, characterised in that the carrier substance is expressly accommodated with the unit of active substance in a mould (12).

4. Bait according to claim 1, characterised in that the fat component is vegetable fat or animal fat.

5. Bait according to claim 1, characterised in that fish meal is used as the additive and lure material.

6. Bait according to claim 1, characterised in that an additional, wax-containing additive is contained in the carrier substance to protect the bait against moisture.

7. Bait according to claim 6, characterised in that the wax-containing additive is paraffin.

8. Bait according to claim 1, characterised in that marking substances are contained in the carrier substance.

9. Method of producing a bait according to claim 1, characterised by the following method steps:

a) liquefying the fat component;

b) admixing the additive;

c) cooling the mixture below the inactivation temperature of the active substance used (if required);

d) injecting the pasty mixture, portion by portion, into a mould until its base is completely covered;

e) introducing a unit of active substance into the mould;

f) filling the mould until the unit of active material is completly covered; and

g) cooling the bait below the solidifying temperature of the mixture, which lies above the operational temperature range.

**10.** Method according to claim 9, characterised in that fishmeal, having a particle size of less than 100 μm, is used as the additive.

**11.** Method according to claim 9, characterised in that liquid paraffin is added to, and mixed with, the liquid fat component prior to the admixing of the additive.

**12.** Method according to claim 9, characterised in that the lure material is added to the mixture of fat component and additive.

**13.** Method according to claim 9, characterised in that the lure material is injected or sprayed onto the finished bait.

**Revendications**

**1.** Appât pour animaux avec un agent actif pharmaceutique, par exemple pour la vaccination contre la rage, l'agent actif étant contenu dans une substance porteuse chargée de graisse qui contient, en plus un appât spécifique, et la composante graisseuse de la substance porteuse présentant un point de fusion compris entre 20 et 60°, **caractérisé en ce** que la substance porteuse contient au moins un additif pour la stabilisation de la forme de l'appât et que les proportions relatives des composantes de graisse/additifs sont choisies de telle façon que le mélange peut être traité sous une forme pâteuse au-delà du point de fusion de la composante de graisse et qu'il n'est pas fragile dans la plage des températures d'utilisation.

**2.** Appât selon la revendication 1, caractérisé en ce que la substance porteuse enferme entièrement sous une forme d'enveloppe (10) une unité d'agent actif (11) emballée séparément.

**3.** Appât selon l'une des revendications 1 et 2, caractérisé en ce que la substance porteuse, conjointement avec l'unité d'agent actif, est placée de manière éjectable dans un moule (12).

**4.** Appât selon la revendication 1, caractérisé en ce que la composante de graisse est constituée de graisse végétale ou animale.

**5.** Appât selon la revendication 1, caractérisé en ce que de la farine de poisson est utilisée comme additif et appât.

**6.** Appât selon la revendication 1, caractérisé en ce que la substance porteuse contient un additif supplémentaire cireux comme protection contre l'humidité.

**7.** Appât selon la revendication 6, caractérisé en ce que l'additif cireux est de la paraffine.

**8.** Appât selon la revendication 1, caractérisé en ce que la substance porteuse contient des substances de marquage.

**9.** Procédé pour la fabrication d'un appât selon la revendication 1, caractérisé par les étapes suivantes :

a) Liquéfaction de la composante de graisse,

b) addition de l'additif,

c) refroidissement du mélange à la température d'inactivation de l'agent actif (si nécessaire),

d) injection par portions du mélange pâteux dans un moule jusqu'à ce que le fond de celui-ci soit entièrement recouvert,

e) mise en place d'une unité d'agent actif dans le moule,

f) remplissage du moule jusqu'à ce que l'agent actif soit entièrement recouvert,

g) refroidissement de l'appât au-dessous de la température de solidification du mélange qui est supérieure à la plage de de température d'utilisation.

**10.** Procédé selon la revendication 9, caractérisé en ce que de la farine de poisson d'une grosseur de grains inférieure à 100 μm est utilisée comme additif.

**11.** Procédé selon la revendication 9, caractérisé en ce que avant l'addition de l'additif, de la paraffine liquide est ajoutée à la composante de graisse liquide et mélangée avec celle-ci.

**12.** Procédé selon la revendication 9, caractérisé en ce que l'appât est ajouté au mélange composante de graisse/additif.

13. Procédé selon la revendication 9, caractérisé en ce que l'appât est projeté ou pulvérisé sur l'appât terminé.